# EUROPÄISCHE PATENTANMELDUNG

(11) **EP 4 458 284 A1**
(43) Veröffentlichungstag der Anmeldung: **06.11.2024**
(21) Anmeldenummer: 23171501.2
(22) Anmeldetag: 04.05.2023
(51) Int. Cl.: A61B 17/285, A61B 18/14, A61B 18/08

(54) **MESSEREINHEIT**

(71) Anmelder: Erbe Elektromedizin GmbH, 72072 Tübingen (DE)
(72) Erfinder: BOB, Felix, 72108 Rotterburg (DE)
(74) Vertreter: Rüger Abel Patentanwälte PartGmbB

(57) **Zusammenfassung**

Ein Instrument (10) mit verbesserter Messereinheit weist ein flaches Messer (21) auf, das in einem Messergehäuse (22) angeordnet ist. Dieses weist einen zur Aufnahme des Messers (21) eingerichteten Kanal (23) auf, an dessen Wänden nach innen gerichtet Vorsprünge (33, 34) vorzugsweise in Gestalt von Noppen ausgebildet sind, zwischen denen das Messer (21) mit Spiel gehalten ist. Ist das Messergehäuse (22) aus Kunststoff ausgebildet, können die Vorsprünge (33, 34) auf einfache Weise beim Umformen erzeugt werden. Wird das Messergehäuse (22) aus einem Halbzeug, beispielsweise einem Blechteil durch ein Umformverfahren, beispielsweise durch einen Stanzbiegeprozess erzeugt, können die den Kanal (23) verengenden aufeinander zuweisenden Vorsprünge (33, 34) erzeugt werden, indem die Wände (29, 30) an ihren Au-ßenseiten mit Vertiefungen versehen werden. Die erfindungsgemäße Messereinheit ist besonders robust gegen Verschmutzungen.

## Beschreibung

Die Erfindung betrifft die Messereinheit eines chirurgischen Instruments als eigenständige Baugruppe oder Bestandteil dieses Instruments.

Häufig weisen chirurgische Instrumente einen zangenartigen Werkzeugteil auf, mit dem Gewebe gegriffen werden kann. Häufig ist diesem Werkzeug ein verschiebbar gelagertes Messer zugeordnet, das dazu dient, von der Zange gegriffenes Gewebe zu durchtrennen.

Beispielsweise offenbart dazu die US 2016/0051316 A1 ein für den laparoskopischen Einsatz ausgebildetes Instrument mit einem länglichen Schaft, an dessen distalem Ende ein Werkzeug der genannten Bauart angeordnet ist.

Das zum Durchtrennen von in dem Werkzeug gefassten Gewebe dienende Messer wird in den Branchen des zangenartigen Werkzeugs in einem seitlich gekrümmten Kanal geführt, um, wenn es in Längsrichtung distal vorgeschoben wird, Gewebe zu durchtrennen.

Die beiden Branchen weisen bestrombare Elektroden auf, um das von dem Instrument gegriffene Gewebe zu erhitzen und damit zu koagulieren. Dabei besteht das Risiko, dass Gewebe an dem Messer festklebt, was von der Messertemperatur abhängt. Um eine zu starke Erhitzung des Messers durch Übertragung von Wärme von den Elektroden auf das Messer zu verhindern, sind in den Flanken des Messerkanals Zähne angeordnet, die das Messer lediglich an in Längsrichtung voneinander beabstandeten kleinen Kontaktflächen berühren.

Weiter offenbart die EP 2 893 886 B1 eine Klingenbaugruppe für ein Einweg-Skalpell mit zugehörigem wiederverwendbarem Skalpellgriff. Der Klinge des Skalpells sind ein Klingenhalter und ein Klingenschutz zugeordnet. Damit der Klingenhalter und der Griff in dem Klingenschutz leicht gleiten können, ist letzterer innen mit einer sich in Längsrichtung erstreckenden Gleitrippe versehen.

Weiter offenbart die US 2022/0331498 A1 eine Antihaftbeschichtung für Schneidelemente elektrochirurgischer Schneidwerkzeuge, wie zum Beispiel in Längsrichtung verschiebbare Messer, die zwischen Branchen gefasstes Gewebe durchtrennen sollen. Eine solche Antihaftstruktur kann beispielsweise an Isolationselementen elektrochirurgischer Schneideinrichtungen vorgesehen sein. Die hydrophoben Strukturen können den Kontakt zwischen dem Gewebe und den Isolationselementen durch mikroskopische Vorsprünge und Vertiefungen in der Oberfläche auf minimale Bereiche beschränken.

Weiter ist aus der EP 3 815 642 A1 eine Messerkartusche für ein Versiegelungsinstrument bekannt, bei dem das in Längsrichtung verschiebbar gelagerte Messer in einer von dem Instrument separierbaren gesonderten Kartusche gelagert ist.

Bei Verwendung eines Instruments am Patienten kann das Messer mehrfach in Längsrichtung bewegt werden, wodurch Gewebeflüssigkeit in den Messerkanal verschleppt werden kann. Wird das Messer dann eine gewisse Zeit nicht betätigt, kann es dazu neigen, festzukleben, sodass es nur schwergängig oder nicht mehr zu bewegen ist. Dies kann den Operationsprozess nachhaltig stören.

Es ist Aufgabe der Erfindung, ein verbessertes Instrument, insbesondere eine verbesserte Messereinheit anzugeben.

Diese Aufgabe wird mit der Messereinheit nach Anspruch 1 gelöst:
Die erfindungsgemäße Messereinheit weist ein Messergehäuse mit einem sich in einer Längsrichtung erstreckenden Kanal zur Aufnahme des Messers auf. Der Kanal ist so lang, dass er das streifenförmige Messer vollständig aufnehmen kann, sodass dieses nicht oder nur wenig aus einem Ende des Kanals heraussteht. Andererseits ist das Messer so lang, dass es zum Durchtrennen von biologischem Gewebe in Längsrichtung so weit aus dem Kanal zum Beispiel in den Messerschlitz von Branchen eines zangenartigen Instruments vorgeschoben werden kann, dass es den gesamten Messerschlitz durchstreift, wobei ein hinterer (proximaler) Teil des Messers noch immer in dem Kanal verbleibt.

Das Messergehäuse kann Bestandteil eines chirurgischen Instruments, einer Messerkartusche oder einer Messerkassette sein, die lösbar mit dem Instrument verbunden ist. Die Kartusche oder Kassette kann nach dem Vorbild der EP 3 815 642 A1 oder auch anderweitig ausgebildet sein. Ist das Messergehäuse Teil des Instruments, geht der Kanal in den genannten Messerschlitz vorzugsweise glatt und stufenlos über. Ist das Messergehäuse hingegen Teil einer Messerkassette oder Messerkartusche, sind an dem Instrument Anschlussmittel vorgesehen, die die Messerkassette oder Messerkartusche so halten, dass der Kanal des Messergehäuses glatt an den Messerschlitz des Instruments anschließt.

Das Messergehäuse weist in seinem Kanal an denjenigen Flächen, die den Flanken des Messers zugewandt sind, Vorsprünge auf. Diese Vorsprünge bewirken, dass immer ein Mindestabstand zwischen den Flächen des Kanals und den Flanken des Messers eingehalten wird. Wird das Messer beim Schneiden von Gewebe in Längsrichtung vorwärts und rückwärts bewegt und werden damit Sekrete, Gewebereste oder dergleichen in den Kanal eingetragen, wird durch den mittels der Vorsprünge garantierten Mindestabstand zwischen den Flächen und den Flanken sichergestellt, dass das Messer nicht in dem Kanal festklebt. Es kann dann auch, wenn das Instrument während der Operation zunächst beiseitegelegt, um nach kurzer oder auch längerer Zeit erneut in Benutzung genommen zu werden, nicht zum Festkleben des Messers und somit zu einer Behinderung der Benutzung des Instruments kommen.

Vorzugsweise ist das Messer in Ruheposition von dem Messergehäuse vollständig aufgenommen. Dadurch liegt die Schneide des Messers geschützt. Außerdem kann die Messereinheit, wenn sie als selbstständige Messerkassette oder Messerkartusche ausgebildet ist, das Verletzungsrisiko für Bedienungspersonal mindern oder beseitigen.

Vorzugsweise ist das Messer in dem Kanal mit Spiel insbesondere mit seitlichem Spiel angeordnet. Das Spiel beträgt vorzugsweise mehrere hundertstel Millimeter, wobei die Höhe der Vorsprünge über der sonstigen Fläche des Kanals vorzugsweise wenigstens ein Zehntelmillimeter beträgt. Auf diese Weise werden Kapillareffekte weitgehend unterbunden, die ansonsten zum leichten Eindringen von Körperflüssigkeiten, Sekreten, insbesondere eiweißhaltigen Flüssigkeiten in den Kanal führen könnten.

Das Messer ist vorzugsweise aus einem Metall, beispielsweise Edelstahl, ausgebildet. Ebenso kann das Messergehäuse vorzugsweise aus einem Metall oder alternativ auch aus einem Kunststoff ausgebildet sein. Die Anordnung der Vorsprünge an den Seitenflächen des Kanals gestattet die Anwendung einfacher und sicherer Fertigungstechnologien. Beispielsweise kann das Messer als Flachteil ohne plastische Verformung, beispielsweise durch geeignete Trennverfahren aus Flachmaterial, hergestellt werden. Demgegenüber kann das Messergehäuse zum Beispiel als Stanzbiegeteil erzeugt werden, wobei die nach innen gerichteten Vorsprünge beispielsweise durch Prägen erzeugt werden können. Das Messergehäuse kann jedoch auch aus Kunststoff ausgebildet werden. Unabhängig davon entsteht zwischen den Vorsprüngen und den Flanken des Messers lediglich eine lokale Berührung, beispielsweise eine Punktberührung oder eine Berührung entlang von Linien, insbesondere in Längsrichtung (Bewegungsrichtung) verlaufenden Linien, vorzugsweise insbesondere unterbrochenen Linien. Die Vorsprünge sind dabei vorzugsweise gerundet und gehen somit stufenlos in die entsprechenden Flächen über. Dies reduziert etwaige Reibung zwischen dem Messer und der Messerführung, insbesondere bei Vorhandensein von Geweberesten oder Sekreten in dem Kanal.

Weitere Einzelheiten vorteilhafter Ausführungsformen der Erfindung ergeben sich aus Unteransprüchen, aus der Beschreibung oder der zugehörigen Zeichnung: In der Zeichnung zeigen:
Figur 1 ein Instrument mit erfindungsgemäßer Messereinheit, in schematisierter perspektivischer Darstellung,
Figur 2 ein Instrument mit abnehmbarer erfindungsgemäßer Messereinheit, in perspektivischer schematisierter Darstellung,
Figur 3 die Messereinheit eines Instruments nach Figur 1 oder 2, in schematisierter perspektivischer Darstellung,
Figur 4 die Messereinheit nach Figur 3, in längsgeschnittener Explosionsdarstellung,
Figur 5 die Messereinheit nach Figur 3, in Querschnittsdarstellung und
Figur 6 die Messereinheit nach Figur 5, in längsgeschnittener ausschnittsweiser Darstellung.

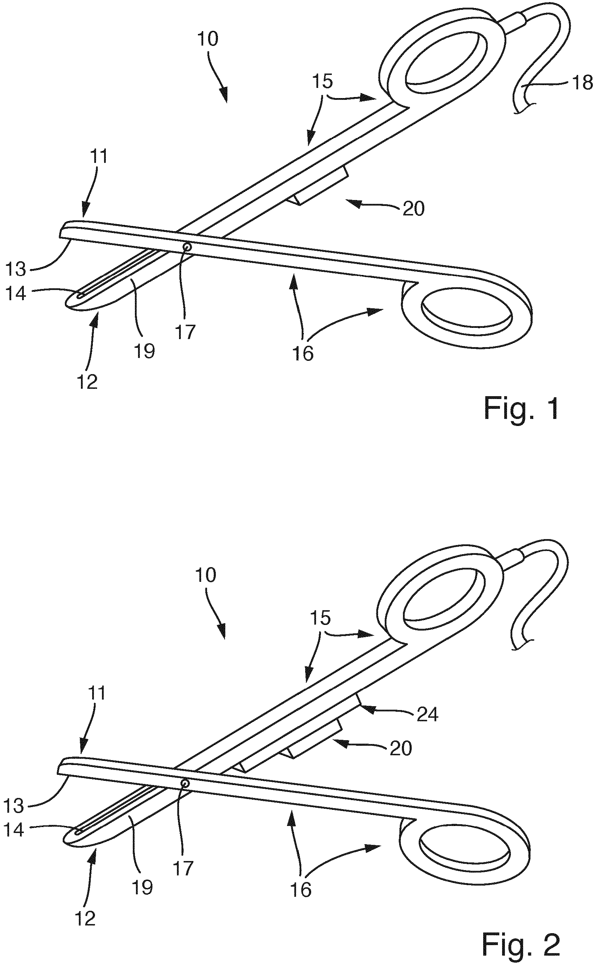

In Figur 1 ist ein Instrument 10 veranschaulicht, das dazu eingerichtet ist, mittels zweier Branchen 11 und 12 Gewebe zu fassen, zu koagulieren und zu trennen. Dazu sind die Branchen 11, 12 an ihren aufeinander zu weisenden Seiten mit Elektroden 13, 14 versehen, um dazwischen gefasstes Gewebe mittels Durchleitung elektrischen Stroms erhitzen und somit koagulieren zu können. Das Öffnen und Schließen der Branchen 11, 12 erfolgt über Handgriffe 15, 16, die in die Branchen 13, 14 übergehen und an einem Scharnier 17 gelenkig miteinander verbunden sind. Die zur Bestromung der Elektroden 13, 14 und somit des Gewebes erforderliche elektrische Leistung kann über ein Kabel 18 von einem Generator herangeführt werden.

In zumindest einer der Branchen 11, 12, vorzugsweise in beiden, ist jeweils ein Messerschlitz 19 angeordnet. Mittels eines geeigneten Betätigungselements, beispielsweise eines an einem der Handgriffe 15, 16 angeordneten Schiebers 20, lässt sich ein Messer 21 in dem Messerschlitz 19 bewegen. Zur Betätigung desselben dient eine geeignete Betätigungseinrichtung, wie beispielsweise der Schieber 20, der an einem der Handgriffe 15, 16 angeordnet ist.

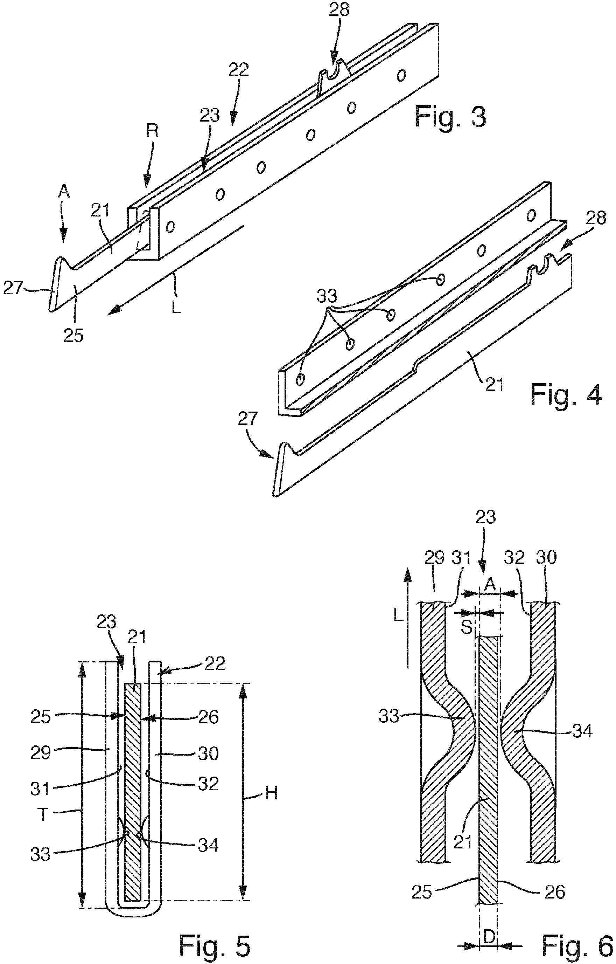

Das Messer 21 ist in den Figuren 3, 4, 5 und 6 in verschiedenen Zusammenhängen gesondert veranschaulicht. Das Messer 21 ist in einem Messergehäuse 22 in einem Kanal 23 angeordnet, der eine Längsrichtung L festlegt. Das Messergehäuse 22 kann Teil des Handgriffs 15 und somit fest mit dem Instrument 10 verbunden sein. Alternativ kann das Messergehäuse 22 Teil einer Messerkassette 24 sein, die an einem der Handgriffe 15, 16 lösbar angeordnet ist, um die Messerkassette 24 und mit ihr das Messer 21 nach Gebrauch austauschen zu können. Ein solches Instrument 10` ist in Figur 2 schematisiert veranschaulicht. Es kann sich dabei insbesondere um ein wiederverwendbares sterilisierbares Instrument 10` handeln, das vor Gebrauch mit einer nicht wiedersterilisierbaren und somit nicht wiederverwendbaren, jedoch steril bereitgestellten Messerkassette 24 verbunden wird.

Zur Betätigung des Messers 21, d.h. zur Verschiebung des Messers 21 in Längsrichtung L, kann wiederum ein Schieber 20 direkt an der Kassette oder irgendein anderes geeignetes Betätigungselement an anderer Stelle des Instruments 10' vorgesehen sein. Ansonsten gilt die im Zusammenhang mit Figur 1 geltende Beschreibung entsprechend für das Instrument 10` nach Figur 2 unter Zugrundelegung der bereits eingeführten Bezugszeichen entsprechend.

Sowohl bei dem Instrument 10 nach Figur 1 als auch bei dem Instrument 10' nach Figur 2 sind das Messer 21 und das Messergehäuse 22 nach den in den Figuren 3 bis 6 veranschaulichten Prinzipien ausgebildet.

Das Messer 21 ist vorzugsweise ein flaches streifenförmiges Blechteil gleichmäßiger Dicke mit zwei Flachseiten, die voneinander weg weisende Flanken 25, 26 bilden. An einem stirnseitigen Ende des Messers 21 ist eine Schneidkante 27 ausgebildet, die quer und somit schräg oder rechtwinklig zu der Längsrichtung L orientiert sein kann. Ansonsten ist das Messer 21 vorzugsweise an beiden Flanken 25, 26 eben und ganz oder zumindest weitgehend unterbrechungsfrei, d.h. ohne Öffnungen, Schlitze oder dergleichen ausgebildet.

An seinem von der Schneidkante 27 abliegenden Ende kann das Messer 21 eine Koppelstruktur 28 aufweisen, um durch einen Betätigungsmechanismus, z.B. in Gestalt des Schiebers 20, gezielt in der Längsrichtung L oder gegen die Längsrichtung L verschoben zu werden. Die Koppelstruktur 28 kann eine maulartige, sich quer zu der Längsrichtung L öffnende Ausnehmung, ein Vorsprung oder dergleichen sein.

Das Messergehäuse 22 kann beispielsweise ein im Querschnitt U-förmiges gebogenes Blechteil, zum Beispiel ein Stanzbiegeteil oder auch ein Kunststoffteil sein. Es weist zwei Wände 29, 30 mit aufeinander zu weisenden Flächen 31, 32 auf. An den Flächen 31, 32 sind Vorsprünge 33, 34 angeordnet, die aufeinander zu weisen und zwischen einander das Messer 21 mit Spiel aufnehmen. Die Vorsprünge 33, 34 können, wie Figur 6 veranschaulicht, durch von außen in die jeweilige Wand 29, 30 eingeprägte Vertiefungen gebildet sein. Diese Vorsprünge 33, 34 bilden einwärts gerichtete Noppen, beispielsweise in Gestalt gerundeter Erhebungen. Zwischen zwei einander gegenüberliegenden Noppen 33, 34 ist ein Abstand A definiert. Dieser ist größer als eine Dicke D des Messers 21, die zwischen seinen Flanken 25, 26 zu messen ist. Die Differenz zwischen dem Abstand A und der Dicke D ist das Spiel S, das vorzugsweise etwa 0,05 mm beträgt. Es kann auch größer, zum Beispiel 0,1 mm oder kleiner, zum Beispiel lediglich 0,03 mm sein. Die Vorsprünge 33, 34 erheben sich vorzugsweise etwa um einen Betrag von 0,1 mm über die jeweilige Fläche 31, 32. Der Abstand A beträgt vorzugsweise etwa 0,2 mm bei einer Dicke D des Messers 21 von zum Beispiel ungefähr 0,15 mm.

Die insoweit beschriebene zumindest aus dem Messer 21 und dem Messergehäuse 22 bestehende Messereinheit arbeitet im Zusammenhang mit dem Instrument 10 oder 10` nach Figur 1 oder 2 wie folgt:

Zum Koagulieren und Trennen von Gewebe, beispielsweise Blutgefäßen oder anderweitigen Gefäßen oder anderweitigem biologischem Gewebe, wird dieses durch Schlie-ßen der Branchen 11, 12 gefasst und mit den Elektroden 13, 14 bestromt, um einen Koagulationseffekt zu bewirken. Ist dieser ausreichend fortgeschritten, kann das Gewebe mittels der Schneidkante 27 des Messers 21 getrennt werden. Das Messer 21 wird dazu durch Betätigung des Schiebers 20 oder eines sonstigen geeigneten Betätigungsorgans zumindest teilweise aus der in Figur 3 gestrichelt dargestellten Ruheposition R in die Aktivposition A überführt. Dabei verlässt der distale Teil des Messers 21 den Kanal 23 und läuft in dem Messerschlitz 19 zwischen den geschlossenen Branchen 11, 12 in Richtung des distalen Endes derselben. Das Messer 21 durchtrennt dabei das Gewebe. Nach Durchführung des Schnitts kann das Messer 21 wieder aus dem Messerschlitz 19 heraus und in den Kanal 23 hinein bewegt werden. Wird das Instrument 10 oder 10' wieder geöffnet, werden die nunmehr voneinander separierten und durch den Koagulationsprozess versiegelten Enden zum Beispiel eines Blutgefäßes freigegeben.

Während einer Operation kann dieser Vorgang mehrfach oder vielfach wiederholt werden. Dabei kann das Messer 21, das mit seinen Flanken 25, 26 biologisches Gewebe berührt hat, Sekret und Gewebe in den Kanal 23 eintragen. Außerdem kann durch Arbeit im feuchten Milieu Flüssigkeit zu dem Messergehäuse 22 gelangen. Die Vorsprünge 33, 34 verhindern jedoch die Ausbildung eines zu engen Spalts zwischen den Flächen 31, 32 und den ihnen gegenüberliegenden Flanken 25, 26 des Messers 21. Es wird die Ausbildung von Kapillarspalten vermieden, die ansonsten ein Eindringen von Flüssigkeiten in den Kanal 23 fördern würden. Außerdem verhindern die Vorsprünge 33, 34 selbst dann, wenn Flüssigkeit in den Kanal 23 eingedrungen ist, dass das Messer 21 schwergängig wird, hängenbleibt oder gar festklebt.

Die Vorsprünge 33, 34 sind aufgrund ihrer sanften Wölbung geeignet, als Abstreifer für an dem Messer vorhandene, fest oder zäh gewordene Gewebeflüssigkeit zu dienen. Dabei ist es insbesondere hilfreich, dass die Vorsprünge 33, wie aus Figur 4 ersichtlich, auf einer gemeinsamen Linie in Längsrichtung L, d.h. in Bewegungsrichtung des Messers 21 auf einer Linie hintereinander liegen, wobei ihre Abmessungen senkrecht zu der Bewegungsrichtung L (in Figur 5, vertikal gesehen) deutlich geringer sind, als die in gleicher Richtung gemessene Tiefe T des Kanals 23 bzw. Höhe H des Messers 21.

Die in der Längsrichtung L voneinander distanzierten Vorsprünge 23, insbesondere in Form von Noppen, sind in der Lage, an den Flanken 25, 26 des Messers 21 haftendes Material beiseite zu schieben und in dem Zwischenraum zwischen der Flanke 25 und der Fläche 31 unschädlich zu sammeln. Gleiches gilt für die Vorsprünge 34 und die Flanke 26, sowie die Fläche 32. Im Ergebnis bleibt das Messer 21 leichtgängig in Längsrichtung bewegbar und zwar auch dann, wenn Gewebeflüssigkeit in den Kanal 23 eingedrungen ist und sich womöglich auch an dem Messer 21 festgesetzt hat. Außerdem wird die Neigung des Eindringens von Flüssigkeit in den Kanal 23 gemindert.

Ein Instrument 10 mit verbesserter Messereinheit weist ein flaches Messer 21 auf, das in einem Messergehäuse 22 angeordnet ist. Dieses weist einen zur Aufnahme des Messers 21 eingerichteten Kanal 23 auf, an dessen Wänden nach innen ragende Vorsprünge 33, 34 vorzugsweise in Gestalt von Noppen ausgebildet sind, zwischen denen das Messer 21 mit Spiel gehalten ist. Ist das Messergehäuse 22 aus Kunststoff ausgebildet, können die Vorsprünge 33, 34 auf einfache Weise beim Umformen erzeugt werden. Wird das Messergehäuse 22 aus einem Halbzeug, beispielsweise einem Blechteil durch ein Umformverfahren, beispielsweise durch einen Stanzbiegeprozess erzeugt, können die den Kanal 23 verengenden aufeinander zuweisenden Vorsprünge 33, 34 erzeugt werden, indem die Wände 29, 30 an ihren Außenseiten mit Vertiefungen versehen werden. Die erfindungsgemäße Messereinheit ist besonders robust gegen Verschmutzungen.

### Bezugszeichen:

- 10, 10`: Instrument
- 11, 12: Branchen
- 13, 14: Elektroden
- 15, 16: Handgriffe
- 17: Scharnier
- 18: Kabel
- 19: Messerschlitz
- 20: Schieber
- 21: Messer
- 22: Messergehäuse
- 23: Kanal
- L: Längsrichtung
- 24: Messerkassette
- 25, 26: Flanken des Messers 21
- 27: Schneidkante
- 28: Koppelstruktur
- 29, 30: Wände des Messergehäuses 22
- 31, 32: Flächen (Innenflächen) der Wände 29, 30
- A: Abstand
- D: Dicke des Messers 21
- S: seitliches Spiel des Messers in dem Kanal 23
- 33, 34: Vorsprünge
- T: Tiefe des Kanals 23
- H: Höhe des Messers 21

## Patentansprüche

1. Messereinheit, insbesondere an oder für ein medizinisches, insbesondere chirurgisches Instrument (10, 10'),
mit einem Messer (21), das einen streifenförmigen Körper mit zwei voneinander weg weisenden Flachseiten (25, 26) aufweist,
mit einem Messergehäuse (22), das einen Kanal (23) aufweist, in dem das Messer (21) in einer Längsrichtung (L) zwischen einer Ruheposition (R) und einer Aktivposition (A) verschiebbar angeordnet ist,
wobei das Messer (21) in der Aktivposition (A) teilweise aus dem Messergehäuse (22) heraus bewegt ist,
wobei das Messergehäuse (22) in seinem Kanal (23) an den den Flanken (25, 26) des Messers (21) zugewandten Flächen (31, 32) Vorsprünge (33, 34) aufweist.

2. Messereinheit nach Anspruch 1, **dadurch gekennzeichnet, dass** das Messer (21) in Ruheposition (R) von dem Messergehäuse (22) vollständig aufgenommen ist.

3. Messereinheit nach einem der vorstehenden Ansprüche, **dadurch gekennzeichnet, dass** das Messer (21) in dem Kanal (23) mit Spiel (S) angeordnet ist.

4. Messereinheit nach einem der vorstehenden Ansprüche, **dadurch gekennzeichnet, dass** der Kanal (23) durch zwei aufeinander zu weisende Flächen (31, 32) begrenzt ist, die ebene Flächen sind.

5. Messereinheit nach einem der vorstehenden Ansprüche, **dadurch gekennzeichnet, dass** das Messer (21) aus einem Metall ausgebildet ist.

6. Messereinheit nach einem der vorstehenden Ansprüche, **dadurch gekennzeichnet, dass** an einem Ende des Messers (21) eine Schneide (27) ausgebildet ist.

7. Messereinheit nach Anspruch 6, **dadurch gekennzeichnet, dass** die Schneide (27) quer zu der Längsrichtung (L) angeordnet ist.

8. Messereinheit nach einem der vorstehenden Ansprüche, **dadurch gekennzeichnet, dass** an dem Messer (21) eine Koppelstruktur (28) ausgebildet ist, die zum Anschluss einer Antriebseinrichtung (20) eingerichtet ist.

9. Messereinheit nach einem der vorstehenden Ansprüche, **dadurch gekennzeichnet, dass** alle Vorsprünge (33) auf einer gemeinsamen in Längsrichtung (L) orientierten geraden Linie angeordnet sind.

10. Messereinheit nach einem der vorstehenden Ansprüche, **dadurch gekennzeichnet, dass** die Vorsprünge (33, 34) gerundete Erhebungen auf den Flächen (31, 32) sind.

11. Messereinheit nach einem der vorstehenden Ansprüche, **dadurch gekennzeichnet, dass** zwischen den Vorsprüngen (33, 34) und den Flanken (25, 26) Linienberührungen oder Punktberührungen ausgebildet sind.

12. Messereinheit nach einem der vorstehenden Ansprüche, **dadurch gekennzeichnet, dass** die Vorsprünge (33, 34) Noppen sind.

13. Messereinheit nach Anspruch 12, **dadurch gekennzeichnet, dass** der Abstand (A) zwischen zwei einander gegenüberliegend angeordneten Noppen um ein Spiel (S) größer ist, als eine zwischen den Flanken (25, 26) des Messers (21) zu messende Dicke (D).

14. Messereinheit nach einem der vorstehenden Ansprüche, **dadurch gekennzeichnet, dass** die Vorsprünge (33, 34) auf einer in Längsrichtung (L) orientierten Linie angeordnet sind.

15. Messereinheit nach einem der vorstehenden Ansprüche, **dadurch gekennzeichnet, dass** das Messergehäuse (22) Teil einer Wechselkartusche (24) ist.
